# EUROPÄISCHE PATENTANMELDUNG

(11) **EP 0 974 313 A1**
(43) Veröffentlichungstag der Anmeldung: **26.01.2000**
(21) Anmeldenummer: 99109445.9
(22) Anmeldetag: 11.05.1999
(51) Int. Cl.: A61F 2/06

(54) **Katheter-Anordnung zur Stent-Implantation**

(30) Priorität: 23.07.1998 DE 19833215
(71) Anmelder: Jomed Implantate GmbH, 72414 Rangendingen (DE)
(72) Erfinder: Schmidt, Rainer, 72654 Neckartenzlingen (DE); Jörgensen, Ib, 72401 Halgerloch (DE); von Oepen, Randolf Dr., 72145 Hirrlingen (DE)
(74) Vertreter: Schmitz, Hans-Werner, Dipl.-Ing.

(57) **Zusammenfassung**

Die Erfindung betrifft eine Katheter-Anordnung (1) zur Stent-Implantation, bei der auf einem Führungsdrahttubus (2) ein Ballon (4) montiert ist. Innerhalb des Ballons (4) sind Röntgenkontrastmarker (7, 8) vorgesehen, auf denen wiederum eine Fixationseinrichtung (9) fixiert ist. Die Fixationseinrichtung (9) dient dazu, den Stent (5) beim Aufkrimpen auf den Ballon sicher zu halten.

## Beschreibung

Die Erfindung betrifft eine Katheter-Anordnung nach dem Oberbegriff des Anspruches 1.

Bei einer bekannten Katheter-Anordnung zur Implantation eines Stents in das Gefäß eines Patienten ist ein Ballon vorgesehen, der auf einem Führungsdrahttubus eines Führungsdrahtes fixiert ist. Damit der Ballon und ein auf diesem fixierter Stent in das Blutgefäß an die Stelle der aufzuweitenden Stenose eingeführt werden kann, ist der Ballon kollabierbar. In diesem Zustand wird der Stent auf dem Ballon durch Aufkrimpen temporär festgelegt, damit er beim Hindurchführen durch das Gefäß bis zur Stelle der Implantation nicht vom Ballon herunterrutscht.

Bei bisher bekannten Katheter-Anordnungen werden innerhalb des Ballons vorgesehene Röntgenkontrastmarker zur Fixierung des Stents benutzt. Hierzu wird der Stent zwischen den Röntgenkontrastmarkern auf den Ballon aufgekrimpt, so daß er sich mit seinen Enden an den Markern während des Einführens in das Gefäß des Patienten abstützen kann. Dies heißt mit anderen Worten, daß die Länge des Stents bei der bekannten Katheter-Anordnung kleiner sein muß, als der Abstand der aufeinander zuweisenden Innenkanten der Röntgenkontrastmarker. Folglicherweise ist die Länge des Ballons größer als diejenige des Stents.

Im Rahmen der Erfindung durchgeführte Untersuchungen haben jedoch ergeben, daß sich hieraus erhebliche Gefahren bzw. Nachteile ergeben. Denn beim Aufweiten des Ballons zum Plazieren des Stents in der Stenose können sich die Ballonenden, die den Stent überragen, an den Rändern der Stenose erheblich aufweiten, so daß die Gefahr eines Einschnittes in die Gefäßwand besteht.

Es ist daher Aufgabe der vorliegenden Erfindung, eine Katheter-Anordnung der im Oberbegriff des Anspruchs 1 angegebenen Art zu schaffen, die einfach und funktionssicher aufgebaut ist und die zuvor erläuterten Gefahren des Einschnittes in die Gefäßwand neben der Stenose vermeidet bzw. erheblich minimiert.

Die Lösung dieser Aufgabe erfolgt durch die Merkmale des Anspruches 1.

Durch das Vorsehen einer Fixationseinrichtung innerhalb des Ballons wird eine Versteifung bzw. Verfestigung des Bereichs zwischen den Röntgenkontrastmarkern erreicht, auf die der Stent aufgekrimpt werden kann. Die genannte Verfestigung durch die Fixationseinrichtung läßt den Aufbau ausreichender Haltekräfte beim Aufkrimpen des Stents auf den Ballon möglich werden, so daß ein Verlieren des Stents beim Hindurchführen durch das Gefäß sicher verhindert werden kann. Dadurch wird ferner erreicht, daß die Ballonlänge der Stentlänge angepaßt werden kann, da die Stentlänge dem Abstand der Außenkanten der Röntgenkontrastmarker gleich oder zumindest annähernd gleich sein kann. Dies wiederum verhindert ein Ausstülpen der Ballonenden beim Aufweiten des Ballons, was wiederum die Gefahr von Einschnitten in die Gefäßwand benachbart zur Stenose zumindest erheblich reduziert wenn nicht gar gänzlich vermeidet.

Die Unteransprüche haben vorteilhafte Weiterbildungen der Erfindung zum Inhalt.

Vom Prinzip her kann als Fixationseinrichtung ein Bauteil verwendet werden, das einerseits die Biegesteifigkeit der Katheter-Anordnung nicht ungebührlich erhöht und andererseits ausreichend fest ist, um die erforderlichen Haltekräfte zur temporären Fixierung des Stents auf dem Ballon aufzubringen. Beispielsweise kann ein biegeweiches Zylinderrohr aus geeignetem Material, wie Kunststoff, Gummi oder Metall vorgesehen sein.

Ferner ist es besonders bevorzugt, als Fixationseinrichtung eine Spirale oder ein Spiralband zu nehmen, das besonders biegeweich ist, andererseits jedoch ausreichend hohe Haltekräfte aufbauen kann.

Weitere Einzelheiten, Merkmale und Vorteile der Erfindung ergeben sich aus nachfolgender Beschreibung eines Ausführungsbeispiels anhand der Zeichnung.

Es zeigt:
- Fig. 1: eine schematisch stark vereinfachte teilweise geschnittene Darstellung einer erfindungsgemäßen Katheter-Anordnung, und
- Fig. 2: eine der Fig. 1 entsprechende stark schematisch vereinfachte Darstellung einer möglichen Ausführungsform einer Verrutschsicherung.

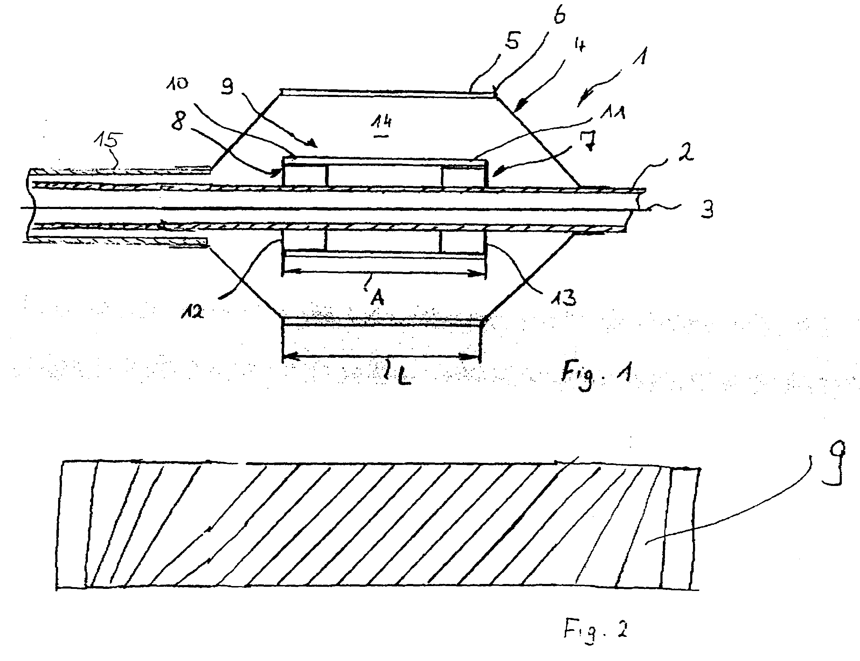

In Fig. 1 ist eine Katheter-Anordnung 1 zur Implantation eines Stents 5 dargestellt. Die Katheter-Anordnung 1 weist im Beispielsfall einen Führungsdrahttubus 2 auf, in dem ein Führungsdraht 3 angeordnet ist, die zusammen eine Führungseinrichtung bilden.

Auf dem Führungsdrahttubus ist ein Ballon 4 in üblicher Art und Weise fixiert. Hierbei ist der Ballon 4 distal auf dem Führungsdrahttubus 2 und proximal auf einem koaxial über dem Führungsdrahttubus verlaufenden Tubus 15 fixiert, wobei unter "distalem Ende" "zum Herzen hinführend" und unter "proximalem Ende" "vom Herzen wegführend" verstanden wird. Der Ballon 4 ist einerseits zum Einführen des Stents 5 in ein Gefäß eines Patienten kollabierbar und andererseits durch Einführen eines geeigneten Mediums, wie beispielsweise Kochsalzlösung oder Röntgenkontrastmittel, aufweitbar, um den Stent 5 an der Stelle der aufzuweitenden Stenose im Gefäß des Patienten implantieren zu können. Fig. 1 zeigt in schematisch stark vereinfachter Darstellung den aufgeweiteten Zustand des Ballons 4.

Im Innenraum 14 des Ballons 4 ist eine Anordnung von zwei Röntgenkontrastmarkern 7 und 8 vorgesehen. Die Röntgenkontrastmarker 7 und 8 sind üblicherweise Ringe, die auf dem durch den Innenraum 14 des Ballons 4 verlaufenden Bereich des Führungsdrahttubus 2 auf diesem fixiert, üblicherweise aufgeklebt oder aufgenietet, sind.

Wie Fig. 1 zeigt, ist der Stent 5 auf der Außenwand 6 des Ballons 4 angeordnet. Um den Stent 5 im kollabierten Zustand des Ballons auf diesem zu fixieren, was üblicherweise als 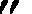 Aufkrimpen" bezeichnet wird, ist eine Fixationseinrichtung 9 vorgesehen. Die Fixationseinrichtung 9 ist bei der dargestellten Ausführungsform auf den Röntgenkontrastmarkern 7, 8 fixiert, beispielsweise aufgeklebt, und liegt im Innenraum 14 des Ballons 4, wie sich dies aus Fig. 1 ergibt.

Der Abstand A der Außenkanten 12, 13 der Röntgenkontrastmarker 7, 8 entspricht hierbei der Länge L des Stents 5. Wie Fig. 1 verdeutlicht, entspricht ferner die Länge der Außenwand 6 des Ballons 4, die den Stent 5 trägt, der Stentlänge L. Dies wird bei der erfindungsgemäßen Katheter-Anordnung dadurch möglich, daß die Fixationseinrichtung 9 ausreichende Haltekräfte für den aufgekrimpten Stent 5 möglich macht, so daß der Stent 5 nicht, wie bei bekannten Anordnungen, zwischen den Röntgenkontrastmarkern 7, 8 im aufgekrimpten Zustand zu liegen kommt, um sich an diesen abstützen zu können. Bei der erfindungsgemäßen Anordnung können somit die Länge der Außenwand 6, die Länge L des Stents 5 und der Abstand A der Außenkanten 12, 13 der Röntgenkontrastmarker 7, 8 gleich oder zumindest annähernd gleich sein, so daß der Stent 5 direkt auf die Röntgenkontrastmarker 7, 8 mit seinen Endbereichen aufgekrimpt werden kann.

Die Fixationseinrichtung 9 kann auf unterschiedliche Art und Weise ausgebildet sein und ist mit ihren Endbereichen 10, 11 auf den Röntgenkontrastmarkern 7, 8 fixiert.

Beispielhaft zeigt Fig. 2 eine spiralartige Ausbildung der Fixationseinrichtung 9, bei der es sich beispielsweise um ein Spiralband handeln kann.

Neben der in den Figuren dargestellten Ausführungsforn ist es im Rahmen der Erfindung ferner möglich, daß der Stent 5 selber mit Röntgenkontrastmarkern versehen ist. In einem solchen Fall kann die Fixationseinrichtung unmittelbar auf den Führungsdrahttubus aufgebracht und auf diesen fixiert werden.

Ferner sind Katheterkonstruktionen bekannt, bei denen ein Teil eines Führungsdrahtes direkt mit dem Kathetern verbunden ist, so daß in einem solchen Falle gemäß dem Prinzip der voliegenden Erfindung die Fixationseinrichtung direkt auf dem Katheter aufgebracht werden kann.

## Patentansprüche

1. Katheter-Anordnung (1) zur Stent-Implantation
- mit einer Führungseinrichtung (2, 3);
- mit einem kollabierbaren und aufweitbaren Ballon (4), der auf der Führungseinrichtung (2, 3) fixiert ist;
- mit einem Stent (5), der auf der Außenwand (6) des Ballons (4) plazierbar ist; und
- mit zumindest zwei Röntgenkontrastmarkern (7, 8)
dadurch gekennzeichnet,
- daß eine Fixationseinrichtung (9) für den Stent (5) im Ballon (4) angeordnet ist.

2. Katheter-Anordnung nach Anspruch 1, dadurch gekennzeichnet, daß die Fixationseinrichtung (9) ein biegeweiches Zylinderrohr ist.

3. Katheter-Anordnung nach Anspruch 1, dadurch gekennzeichnet, daß die Fixationseinrichtung (9) eine Spirale bzw. ein Spiralband ist.

4. Katheter-Anordnung nach einem der Ansprüche 1 bis 3, dadurch gekennzeichnet, daß die Fixationseinrichtung (9) mit ihren Endbereichen (10, 11) auf den Röntgenkontrastmarkern (7, 8) fixiert ist.

5. Katheter-Anordnung nach einem der Ansprüche 1 bis 4, dadurch gekennzeichnet, daß die Länge (L) des Stents (5) zumindestens annähernd gleich dem Abstand (A) der Außenkanten (12, 13) der Röntgenkontrastmarker (7, 8) ist.

6. Katheter-Anordnung nach einem der Ansprüche 1 bis 5, dadurch gekennzeichnet, daß die Führungseinrichtung einen Führungsdrahttubus (2) und einen Führungsdraht (3) aufweist, der durch den Führungsdrahttubus (2) hindurchführbar ist.

7. Katheter-Anordnung nach einem der Anssprüche 1 bis 6, dadurch gekennzeichnet, daß die Röntgenkontrastmarker (7, 8) beabstandet voneinander innerhalb des Ballons (4) auf dem Führungsdrahttubus (2) fixiert sind.

8. Katheter-Anordnung nach Anspruch 1, dadurch gekennzeichnet, daß der Stent (5) die Röntgenkontrastmarker aufweist.
